(19) Europäisches Patentamt
European Patent Office
Office européen des brevets

(11) Veröffentlichungsnummer: **0 001 062**
**B1**

(12) # EUROPÄISCHE PATENTSCHRIFT

(45) Veröffentlichungstag der Patentschrift: **29.04.81**

(51) Int. Cl.³: **C 07 D 457/12,**
//A61K31/48

(21) Anmeldenummer: **78100701.8**

(22) Anmeldetag: **18.08.78**

(54) **Ergolin-Derivate, Verfahren zu ihrer Herstellung, diese enthaltende pharmazeutische Zusammensetzungen und ihre Anwendung bei therapeutischen Behandlungen.**

(30) Priorität: **02.09.77 CH 10732/77**

(43) Veröffentlichungstag der Anmeldung:
**21.03.79 Patentblatt 79/6**

(45) Bekanntmachung des Hinweises auf die Patenterteilung:
**29.04.81 Patentblatt 81/17**

(84) Benannte Vertragsstaaten:
**BE CH DE FR GB LU NL SE**

(56) Entgegenhaltungen:
**FR - A - 2 267 781**

(73) Patentinhaber: **SANDOZ AG**
**Lichtstrasse 35**
**CH-4002 Basel (CH)**

(72) Erfinder: **Hauth, Hartmut, Dr.**
**Burgstrasse 2**
**CH-4125 Riehen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Ergolin-Derivate, Verfahren zu ihrer Herstellung, diese enthaltende pharmazeutische Zusammensetzungen und ihre Anwendung bei therapeutischen Behandlungen

Die Erfindung betrifft eine Verbindung der Formel I,

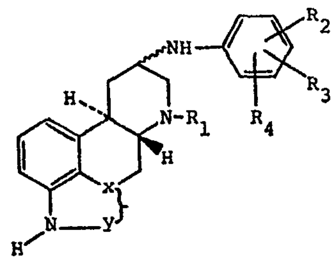

I

worin $R_1$ Wasserstoff oder niederes Alkyl bedeutet, $R_2$, $R_3$ und $R_4$ unabhängig voneinander für Wasserstoff, Halogen, niederes Alkoxy, niederes Alkyl oder

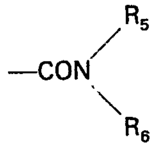

(worin $R_5$ und $R_6$ je Wasserstoff oder niederes Alkyl bedeuten), x y für —CH=C= stehen.

Erfindungsgemäss gelangt man zu einer Verbindung der Formel I, indem man eine Verbindung der Formel II,

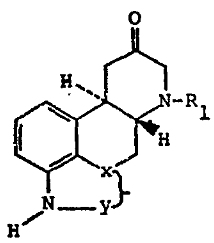

II

worin $R_1$ und x y obige Bedeutung besitzen, in Gegenwart eines Amins der Formel III,

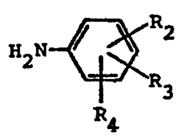

III

worin $R_2$, $R_3$ und $R_4$ obige Bedeutung besitzen, reduziert und aus dem erhaltenen Isomerengemisch einer Verbindung der Formel I eine Verbindung der Formel I a bzw. I b,

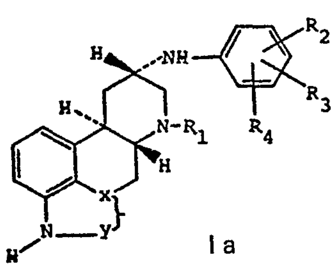

I a

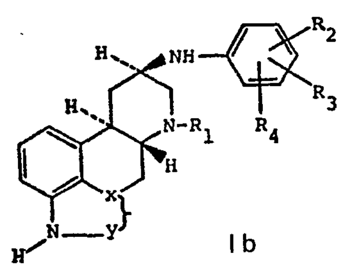

I b

worin jeweils $R_1$ bis $R_4$ und x y obige Bedeutung besitzen, isoliert.

In der Formel I weisen Alkyl oder Alkoxygruppen 1 bis 4 Kohlenstoffatome, insbesondere 1 bis 2 Kohlenstoffatome auf. Halogen steht für Fluor, Brom oder Chlor. $R_1$ steht vorzugsweise für Methyl. $R_2$

befindet sich vorzugsweise in para-Stellung. $R_2$ steht vorzugsweise für Alkoxy oder Halogen. $R_3$ und $R_4$ stehen vorzugsweise für Wasserstoff. Das Verfahren wird in Analogie zu bekannten Methoden ausgeführt. Für die Reduktion vorteilhaft geeignet erwies sich die katalytische Hydrierung in Gegenwart eines Edelmetallkatalysators, vorzugsweise Palladiummetall auf Aktivkohle; man arbeitet vorzugsweise in Eisessig bei Normalbedingungen.

Die Auftrennung des erhaltenen Isomerengemisches kann nach für analoge Verbindungen an sich bekannten Methoden, z.B. durch Chromatographie mit einem geeigneten Lösungsmittelgemisch, wie Methylenchlorid/Methanol erfolgen. Die Verbindungen der Formel I können in freier Form als Base oder in Form von Additionssalzen mit Säuren vorliegen. Aus den freien Basen lassen sich in bekannter Weise Säureadditionssalze herstellen und umgekehrt. Geeignete Salze schliessen das Maleinat und das Hydrochlorid ein.

Die Ausgangsprodukte sind bekannt oder nach an sich bekannten Methoden aus bekannten Verbindungen herstellbar.

Die Verbindungen der Formel I zeigen an Tieren pharmakologische Eigenschaften. Insbesondere zeigen die Verbindungen der Formel I eine zentrale dopaminerge stimulierende Wirkung, wie sie durch Standard-Tests nachgewiesen werden kann. Beispielsweise kann nach der Methode nach U. Ungerstedt, Acta physiol. scand. Suppl. *367*, 69—93 (1973) an Ratten, bei denen durch eine 6-Hydroxydopamin-Injektion in die substantia nigra eine unilaterale Verletzung der nigro-neostriatalen Dopaminbahn erzeugt wurde, mit Dosen zwischen etwa 1 bis 40 mg/kg s.c. eine Rotation in Richtung der nicht denervierten Seite festgestellt werden. Die Verbindungen können daher zur Behandlung von Parkinsonismus Anwendung finden.

Weiterhin zeigen insbesondere die Verbindungen der Formel I a eine Prolactin-Sekretionshemmung. Diese kann beispielsweise an Rattan durch Hemmung der Ei-Implantation wie folgt gezeigt werden:

Die zu untersuchende Verbindung wird weiblichen Ratten 5 Tage nach der Begattung verabreicht, wobei gezeigt wird, dass diese entsprechend dem vaginalen Schmiertest Samen-positiv ist. Die Ratten werden am 12. Tag getötet und ihre Uteri zum Nachweis, dass der Nidationsprozess unterbrochen wurde, mittels der Salewski Reaktion untersucht [Arch. exp. Path. Pharm. *247*, 367 (1967)].

Die Verbindungen werden s.c. verabreicht in Dosen zwischen etwa 1 bis 10 mg/kg Tiergewicht.

Die Verbindungen sind daher indiziert zur Anwendung als Prolactin-sekretionshemmende Mittel.

Für oben genannte Anwendungen liegt die zu verwendende Tagesdosis im Bereich von ungefähr 0,5 bis 50 mg, zweckmässig verabreicht in Teildosen 2 bis 4 mal täglich, in einer Dosierungsform, enthaltend etwa 0,1 bis 25 mg der Substanz, oder in einer geeigneten Form mit verspäteter Wirkstoffabgabe.

Die neuen Verbindungen der Formel I bzw. ihre physiologisch verträglichen Säureadditionssalze können als Arzneimittel allein oder in entsprechende Heilmittel verwendet werden.

Die Erfindung umfasst auch pharmazeutische Zusammensetzungen, die eine Verbindung der Formel I zusammen mit einer Trägersubstanz oder einem Verdünnungsmittel enthalten. Solche pharmazeutische Zusammensetzungen, beispielsweise eine Lösung oder eine Tablette können nach an sich bekannten Methoden hergestellt werden. Die in Beispiel 1 beschriebenen Verbindungen zeigen interessante Wirkungen.

In den nachfolgenden Beispielen erfolgen alle Temperaturangaben in Celsiusgraden.

## Beispiel 1

*8-(4-Aethoxy-phenyl)amino-6-methyl-ergolin*

Zu 17,8 g 10% Palladium auf Aktivkohle und 10,3 g 4-Aethoxyanilin in 300 ml Eisessig wird unter gleichzeitiger Hydrierung unter Normalbedingungen langsam eine Lösung von 17,8 g 6-Methyl-8-oxo-ergolin in 800 ml Eisessig zugetropft. Dauer der Zugabe ca. 8 Stunden. Nach beendeter Wasserstoffaufnahme wird vom Katalysator abfiltriert, das Filtrat im Vakuum eingeengt und der verbleibende Rückstand in Methylenchlorid (+15% Isopropanol) aufgenommen und mit eisgekühltem Ammoniak und Wasser gewaschen. Der nach Trocknen der vereinigten organischen Phasen und Eindampfen verbleibende Rückstand wird zur Reinigung und Trennung der Isomeren an der 80-fachen Menge Kieselgel chromatographiert mit Methylenchlorid unter Zusatz steigender Mengen von Methanol.

Durch Elution mit Methylenchlorid unter Zusatz von 2% Methanol wird das Isomere I a erhalten:

1 a) *(5R,8S,10S)-8-(4-Aethoxy-phenyl)amino-6-methyl-ergolin*
Maleinat:
Smp. 219—222°
$[\alpha]_D^{20} = -25°$ (c=0,48; 50% EtOH)

Durch weitere Elution mit Methylenchlorid unter Zusatz von 4% Methanol wird das Isomere I b erhalten:

1 b) *(5R,8R,10S)-8-(4-Aethoxy-phenyl)amino-6-methyl-ergolin*
Smp. >272° (Zers.)
$[\alpha]_D^{20} = -55°$ (c=0,5; Pyridin)

Analog Beispiel 1 werden die folgenden Verbindungen erhalten:

2 a) *(5R,8S,10S)-8-(4-Fluor-phenyl)amino-6-methyl-ergolin*
Dihydrochlorid:
Smp. 229° (Zers.)
$[\alpha]_D^{20} = -27{,}4°$ (c=0,5 in 50% EtOH)

2 b) *(5R,8R,10S)-8-(4-Fluor-phenyl)amino-6-methyl-ergolin*
Hydrogenmaleinat:
Smp. >229° (Zers.)
$[\alpha]_D^{20} = -46°$ (c=0,5 in $EtOH/H_2O[1:1]$)

3 a) *(5R,8S,10S)-8-(3-N,N-Dimethylcarboxamid-phenyl)amino-6-methyl-ergolin*
Maleinat:
Smp. 216—218°
$[\alpha]_D^{20} = -40°$ (c=0,5; 50% EtOH)

3 b) *(5R,8R,10S)-8-(3-N,N-Dimethylcarboxamid-phenyl)amino-6-methyl-ergolin*
Maleinat:
Smp. 228—230°
$[\alpha]_D^{20} = -36°$ (c=0,5; 50% EtOH)

**Patentansprüche**

1. Verbindungen der Formel

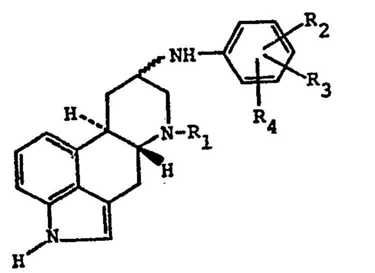

I

worin $R_1$ Wasserstoff oer niederes Alkyl bedeutet, $R_2$, $R_3$ und $R_4$ unabhängig voneinander für Wasserstoff, Halogen, niederes Alkoxy, niederes Alkyl oder

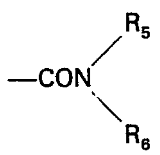

(worin $R_5$ und $R_6$ je Wasserstoff oder niederes Alkyl bedeuten), und ihre Additionssalze mit Saüren.

2. Verbindungen gemäss Anspruch 1, worin $R_1$ Wasserstoff oder Methyl bedeutet.

3. (5R,8S,10S)- oder (5R,8R,10S)-8-(4-Aethoxy-phenyl)amino-6-methyl-ergolin und ihre Additionssalze mit Säuren.

4. (5R,8S,10S)- oder (5R,8R,10S)-8-(4-Fluor-phenyl)amino-6-methyl-ergolin und ihre Additionssalze mit Säuren.

5. (5R,8S,10S)- oder (5R,8R,10S)-8-(3-N,N-Dimethyl-carboxamid-phenyl)amino-6-methyl-ergolin und ihre Additionssalze mit Säuren.

6. Verfahren zur Herstellung der Verbindungen nach Anspruch 1, dadurch gekennzeichnet, dass man eine Verbindung der Formel

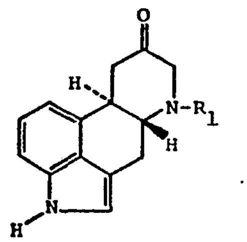

II

worin $R_1$ die im Anspruch 1 genannte Bedeutung besitzt, in Gegenwart eines Amins der Formel

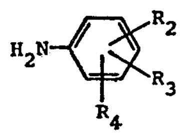

III

worin $R_2$, $R_3$ und $R_4$ die im Anspruch 1 genannte Bedeutung besitzen, reduziert und aus dem erhaltenen Isomerengemisch einer Verbindung der Formel I eine Verbindung der Formel

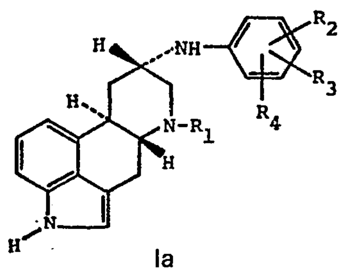

bzw.

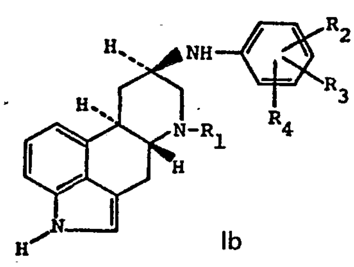

Ia Ib

worin jeweils $R_1$ bis $R_6$ die im Anspruch 1 genannte Bedeutung besitzen, isoliert und gegebenenfalls in ihre Säureadditionssalze überführt.

7. Verfahren nach Anspruch 6, dadurch gekennzeichnet, dass eine Verbindung der Formel II, worin $R_1$ Wasserstoff oder Methyl bedeutet, benutzt wird.

8. Pharmazeutische Zusammensetzungen, dadurch gekennzeichnet, dass sie eine Verbindung gemäss einem der Ansprüche 1 bis 5 in Form der Base oder eines pharmazeutisch verträglichen Säureadditionssalzes enthalten.

9. Pharmazeutische Zusammensetzungen, dadurch gekennzeichnet, dass sie eine Verbindung gemäss einem der Ansprüche 2, 3 oder 4 in Form der Base oder eines pharmazeutisch verträglichen Säureadditionssalzes enthalten.

10. Eine Verbindung gemäss einem der Ansprüche 1 bis 5 in Form der Base oder eines pharmazeutisch verträglichen Säureadditionssalzes zur Anwendung bei der therapeutischen Behandlung.

11. Eine verbindung gemäss Anspruch 10 zur Anwendung bei der Behandlung des Parkinsonismus oder als Prolactinsekretionshemmer.

**Claims**

1. Compounds of formula I

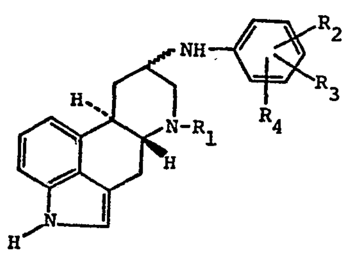

wherein $R_1$ is hydrogen or lower alkyl, $R_2$, $R_3$ and $R_4$ are, independently, hydrogen, halogen, lower alkoxy, lower alkyl or $CONR_5R_6$ (wherein $R_5$ and $R_6$ are, independently, hydrogen or lower alkyl), and acid addition salts thereof.

2. Compounds according to claim 1 wherein $R_1$ is hydrogen or methyl.

3. (5R,8S,10S) or (5R,8R,10S)-8-(4-ethoxy-phenyl)amino-6-methylergoline and acid addition salts thereof.

4. (5R,8S,10S) or (5R,8R,10S)-8-(4-fluorophenyl)amino-6-methylergoline and acid addition salts thereof.

5. (5R,8S,10S) or (5R,8R,10S)-8-(3-N,N-dimethylcarboxamidophenyl)amino-6-methylergoline and acid addition salts thereof.

6. Processes for the production of compounds of claim 1 characterised in that one reduces a compound of formula II

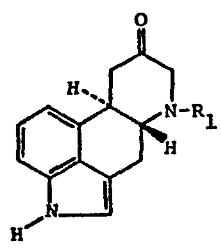

II

wherein $R_1$ is as defined in claim 1 in the presence of an amine of formula III

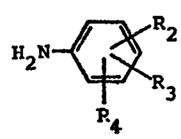

III

wherein $R_2$, $R_3$ and $R_4$ are as defined in claim 1, and from the resulting isomeric mixture isolates a compound of formula

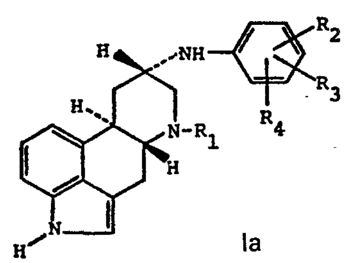

Ia

or

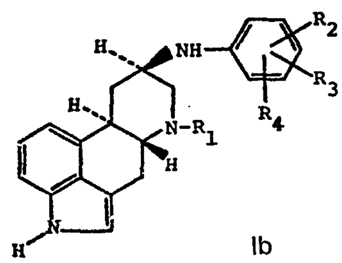

Ib

wherein $R_1$ to $R_4$ are as defined in claim 1, and optionally converting the compound into an acid addition salt.

7. Process according to claim 6 wherein a compound of formula II wherein $R_1$ is hydrogen or methyl is used.

8. Pharmaceutical compositions containing a compound according to any one of claims 1 to 5 in free base form or in pharmaceutically acceptable acid addition salt form.

9. Pharmaceutical compositions containing a compound according to any one of claims 2 to 4 in free base form or in pharmaceutically acceptable acid addition salt form.

10. A compound according to any one of claims 1 to 5 in free base form or in pharmaceutically acceptable acid addition salt form for use in therapy.

11. A compound according to claim 10 for use for the treatment of Parkinsonism or as a prolactin secretion inhibitor.

**Revendications**

1. Composés de formule

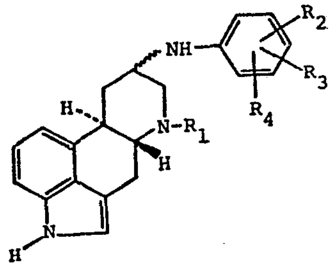

I

dans laquelle $R_1$ signifie l'hydrogène ou un groupe alkyle inférieur, $R_2$, $R_3$ et $R_4$ représentent, indépendamment les uns des autres, l'hydrogène, un halogène, un groupe alcoxy inférieur, alkyle inférieur ou

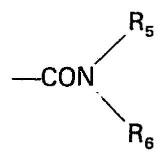

(où $R_5$ et $R_6$ signifient chacun l'hydrogène ou un groupe alkyle inférieur), et leurs sels d'addition avec des acides.

2. Composés selon la revendication 1, dans lesquels $R_1$ signifie l'hydrogène ou un groupe méthyle.

3. (5R,8S,10S)- ou (5R,8R,10S)-8-(4-éthoxyphényl)amino-6-méthyl-ergoline et leurs sels d'addition avec des acides.

4. (5R,8S,10S)- ou (5R,8R,10S)-8-(4-fluorophényl)amino-6-mèthyl-ergoline et leurs sels d'addition avec des acides.

5. (5R,8S,10S)- ou (5R,8R,10S)-8-(3-N,N-diméthyl-carboxamido-phényl)amino-6-méthyl-ergoline et leurs sels d'addition avec des acides.

6. Procédé de préparation des composés selon la revendication 1, caractérisé en ce que l'on réduit un composé de formule

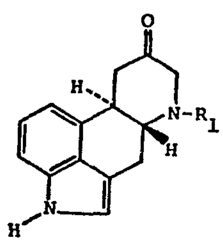

(II)

dans laquelle $R_1$ possède la signification indiquée à la revendication 1, en présence d'une amine de formule

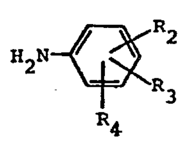

(III)

dans laquelle $R_2$, $R_3$ et $R_4$ possèdent la signification indiquée à la revendication 1 et, à partir du mélange obtenu d'isomères d'un composé de formule I, on isole un composé de formule Ia

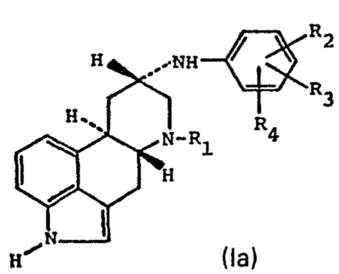

ou

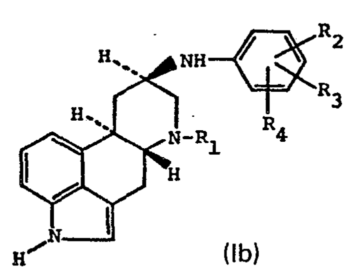

(Ia) (Ib)

dans lesquelles $R_1$ à $R_6$ ont respectivement la signification indiquée à la revendication 1, et, éventuellement, on le transforme en ses sels d'addition avec des acides.

7. Procédé selon la revendication 6, caractérisé en ce que l'on utilise un composé de formule II dans laquelle $R_1$ signifie l'hydrogène ou un groupe méthyle.

8. Compositions pharmaceutiques, caractérisées en ce qu'elles contiennent un composé selon l'une des revendications 1 à 5 sous forme de base ou d'un sel d'addition d'acides acceptable du point de vue pharmaceutique.

9. Compositions pharmaceutiques, caractérisées en ce qu'elles contiennent un composé selon l'une des revendications 2, 3 ou 4 sous forme de base ou d'un sel d'addition d'acides acceptable du point de vue pharmaceutique.

10. Un composé selon l'une des revendications 1 à 5 sous forme de base ou d'un sel d'addition d'acides acceptable du point de vue pharmaceutique pour l'utilisation dans le traitement thérapeutique.

11. Un composé selon la revendication 10 pour l'utilisation dans le traitement de la maladie de Parkinson ou comme inhibiteur de la sécrétion de la prolactine.